# EUROPEAN PATENT SPECIFICATION

(11) **EP 1 907 540 B1**
(45) Date of publication and mention of the grant of the patent: **19.12.2012**
(21) Application number: 06800183.3
(22) Date of filing: 21.07.2006
(51) Int. Cl.: C12N 9/64

(54) **IN-SOLUTION ACTIVATION OF FACTOR VII**
AKTIVIERUNG VON FAKTOR VII IN DER FLÜSSIGEN PHASE
ACTIVATION EN SOLUTION DU FACTEUR VII

(30) Priority: 22.07.2005 US 702041 P
(43) Date of publication of application: 09.04.2008
(73) Proprietor: Bayer HealthCare LLC, Tarrytown, NY 10591 (US)
(72) Inventor: KREBBER, Claus, M., Palo Alto, CA 94303 (US); VISWANATHAN, Sridhar, Menlo Park, CA 94025 (US)
(74) Representative: Carpmaels & Ransford
(86) International application number: PCT/US2006/028285
(87) International publication number: WO 2007/013993

(56) References cited:
- TOMOKIYO K ET AL: "Large-scale production and properties of human plasma-derived activated Factor VII concentrate" VOX SANGUINIS, S. KARGER AG, BASEL, CH, vol. 84, no. 1, January 2003 (2003-01), pages 54-64, XP002286502 ISSN: 0042-9007
- PEDERSEN A H ET AL: "AUTOACTIVATION OF HUMAN RECOMBINANT COAGULATION FACTOR VII" BIOCHEMISTRY, vol. 28, no. 24, 1989, pages 9331-9336, XP002408656 ISSN: 0006-2960 cited in the application
- BJOERN AND L THIM S ET AL: "Activation of coagulation factor VII to VIIa (S Bjoern and L Thim, Novo Research Institute)" RESEARCH DISCLOSURE, MASON PUBLICATIONS, HAMPSHIRE, GB, vol. 269, no. 60, September 1986 (1986-09), XP007110994 ISSN: 0374-4353
- JURLANDER B ET AL: "RECOMBINANT ACTIVATED FACTOR VII (RFVIIA): CHARACTERIZATION, MANUFACTURING, ND CLINICAL DEVELOPMENT" SEMINARS IN THROMBOSIS AND HEMOSTASIS, STUTTGART, DE, vol. 27, no. 4, August 2001 (2001-08), pages 373-383, XP008005254 ISSN: 0094-6176

## Description

### FIELD OF THE INVENTION

The present invention is directed to a method for activation of Factor VII to FVIIa in solution.

### BACKGROUND OF THE INVENTION

Factor VII (FVII), an important protein in the blood coagulation cascade, is a vitamin K-dependent plasma protein synthesized in the liver and secreted into the blood as a single-chain glycoprotein with a molecular weight of 53 kDa. The FVII precursor (sometimes referred to as "single-chain FVII", or scFVII) is converted into an activated form (FVIIa) by proteolytic cleavage at a single site, R152-I153, resulting in two chains linked by a single disulfide bridge. Recombinant human FVIIa is commercially available from Novo Nordisk under the name NovoSeven® and is used for the treatment of bleeding episodes, e.g. in hemophilia or trauma. Recombinantly produced variants of human FVII have also been reported.

Conversion of Factor VII to active Factor VIIa may be achieved using Factor XIIa as described by Hedner and Kisiel (J. Clin. Invest. 71: 1836-1841, 1983), or another protease having trypsin-like specificity (Kisiel and Fujikawa, Behring Inst. Mitt. 73: 29-42, 1983).

Activation of FVII to FVIIa may also be accomplished without the use of added protease by employing the FVII itself, which has an autoproteolytic activity via its serine protease domain. Such "autoactivation" has been accomplished by contacting FVII with a positively-charged surface or resin, such as an anion-exchange resin (Pedersen A. H. et al. Biochemistry 28: 9331-9336, 1989). When performed for example in a column format, active FVIIa may be released from the positively-charged surface or resin by, for example, increasing the ionic strength, decreasing the pH, or by increasing the concentration of Ca²⁺ in the buffer (Bjoern et al., Research Disclosures 269: September 1986, pp. 564 - 565).

Although the use of a positively charged surface or resin for activation of FVII to FVIIa (sometimes referred to as "solid-phase" or "on-column" activation) avoids the use of extrinsically-added protease or tissue factor, there are disadvantages to such method. Since the intrinsic FVII protease activity may also lead to further autodegradation of FVIIa, careful monitoring of critical process parameters are essential to limit the formation of product-related degradation products. However, activation employing a positively charged surface or resin, such as via an ion-exchange column, is highly dependent upon a variety of interdependent factors which are difficult to optimize. Furthermore, such solid-phase or on-column activation processes are not amenable to straightforward scaling up (e.g., for manufacturing) or to scaling down (e.g., for small scale optimization and testing of multiple parameters).

There is a need for an efficient, reliable, reproducible method to activate FVII to FVIIa in solution, without the use of extrinsic protease, tissue factor, or phospholipid, which is amenable to changes in scale and is readily optimized, and leads to improved quality of the final activated protein product. The in-solution activation method described herein fulfills this need.

### SUMMARY OF THE INVENTION

We have explored the possibilities of activating FVII to FVIIa in solution, that is, without contacting the FVII with a solid surface such as an ion-exchange resin to enhance activation, in the absence of added protease, tissue factor, or phospholipid, and it has surprisingly been found that excellent results are obtained using this in-solution activation method.

Thus, the present invention provides a method for activating FVII to FVIIa in solution, comprising: obtaining a solution comprising a substantially purified preparation of scFVII; adding to the solution an amine compound, Ca²⁺ to a final concentration of about 5 mM to about 50 mM (such as about 10 mM to about 30 mM), and adjusting the final pH of the solution to about 7.2 to 8.6 (such as about 7.6 to about 8.2); incubating the resulting activation mixture at between about 2°C and about 25°C for an amount of time sufficient to convert at least 90% of the scFVII to FVIIa; and, optionally, isolating the FVIIa from the activation mixture. The solution comprising the substantially purified preparation of scFVII contains, for example, at least 80% scFVII relative to FVIIa or other FVII-derived fragments, such as, e.g., at least 85%, at least 90%, at least 92%, at least 95%, or at least 98% scFVII relative to FVIIa or other FVII-derived fragments.

In one embodiment, the amine compound is selected from Tris, lysine, arginine, phosphorylcholine, or betaine. In some instances, the amine compound is added to a final concentration of about 50 mM to about 500 mM (such as about 100 mM) in the activation mixture. In some instances a buffer such as borate or HEPES is included to adjust the pH of the activation mixture to the desired pH.

In one embodiment, the activation mixture has an initial concentration of scFVII of at least 4 mg/ml, such as, for example, between about 4 mg/ml and 10 mg/ml. In another embodiment, the activation mixture has an initial concentration of scFVII of about 1 mg/ml to 4 mg/ml, and the activation mixture further comprises an activation enhancer. Some such activation enhancers include polyethylene glycol, glycerol, and ethylene glycol, such as, for example, PEG4000, PEG8000, or glycerol. In some instances, PEG4000 or PEG8000 is present at between about 1% and 10% (w/v) in the activation mixture, such as, for example, 5% PEG4000 or 5% PEG8000. In some instances, glycerol or ethylene glycol is present at between about 5% and 15% (v/v) in the activation mixture, such as, for example, 10% glycerol or 10% ethylene glycol.

In another embodiment, the components of the activation mixture are adjusted such that the amount of time sufficient to convert at least 90% of the scFVII to FVIIa is between about 4 hours and 24 hours at cold room temperature (e.g., about 2°C to 8°C, such as about 4°C) or room temperature (e.g., about 18°C to 25°C, such as about 20°C or 22°C). In some instances, the components of the activation mixture are adjusted such that the amount of time sufficient to convert at least 90% of the scFVII to FVIIa is between about 8 hours and 24 hours at cold room temperature (about 2°C to 8°C, such as about 4°C), such as between about 8 hours and 16 hours.

In one embodiment, the FVII is a FVII variant which differs in 1-15 amino acid residues from the amino acid sequence of wild-type human FVII (SEQ ID NO:1). In another embodiment, the FVII variant comprises the substitutions P10Q or K32E. In another embodiment, the FVII variant comprises the substitutions P10Q and K32E. In another embodiment, the FVII variant comprises the substitutions P10Q, K32E, T106N and V253N.

In another embodiment, the present invention provides a method for activating FVII to FVIIa in solution, comprising obtaining a solution comprising a substantially purified preparation of scFVII; adding Tris or lysine to the solution to a final concentration of about 0.1 M; adding Ca²⁺ to a final concentration of about 5 mM to 50 mM (such as about 10 mM to 30 mM); adjusting the final pH of the solution to about pH 8, wherein the concentration of scFVII is about 4 mg/ml to about 10 mg/ml; incubating the resulting activation mixture at cold room temperature (e.g., about 4°C) for about 8 hrs to about 24 hrs (such as about 8 hrs to 16 hrs); and optionally, isolating the FVIIa from the activation mixture.

In another embodiment, the present invention provides a method for activating FVII to FVIIa in solution, comprising obtaining a solution comprising a substantially purified preparation of scFVII; adding Tris or lysine to the solution to a final concentration of about 0.1 M, adding Ca²⁺ to a final concentration of about 5 mM, adding either PEG4000, PEG8000 or glycerol to about 5%, 5% or 10% respectively, adjusting the final pH of the solution to about 8.0, wherein the concentration of scFVII is about 1 mg/ml to about 4 mg/ml; incubating the resulting activation mixture at cold room temperature (e.g., about 4°C) for about 8 hrs to about 24 hrs; and optionally, isolating the FVIIa from the activation mixture.

These and other objects and features of the invention will become more fully apparent when the following detailed description is read in conjunction with the accompanying figures.

### BRIEF DESCRIPTION OF THE DRAWINGS

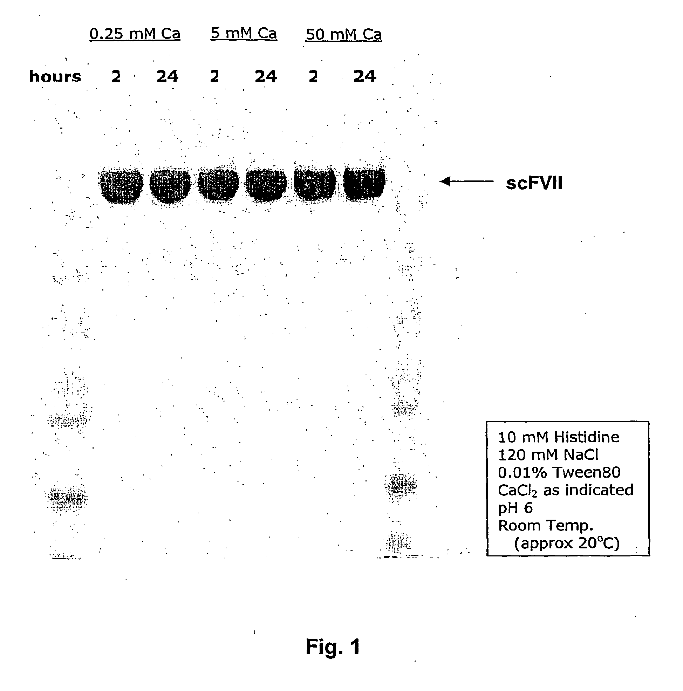

Figure 1 shows an SDS-PAGE gel of a substantially purified preparation of scFVII prepared according to the Materials and Methods, following incubuation for the indicated times at room temperature (approximately 20°C) in 10 mM histidine, 120 mM NaCl, 0.01 % Tween 80 with 0.25 mM to 50 mM CaCl₂ as indicated, pH 6. Densitometery indicates that the preparation is at least 95% scFVII (i.e., less than 5% FVII or other FVII-derived fragments) and that the scFVII preparation does not undergo detectable autoactivation or degradation under the conditions shown.

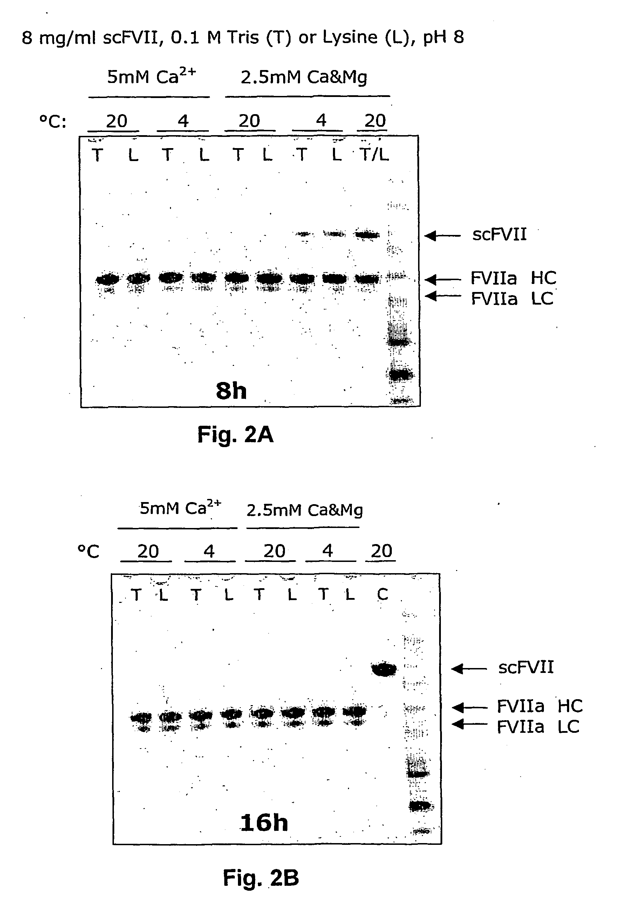

Figures 2A & 2B show in-solution activation to FVIIa of a substantially purified preparation of scFVII. To the 8 mg/ml scFVII preparation (shown in lane C in Fig. 2B) either Tris (T), lysine (L), or both Tris plus lysine (T/L) was added to a final total concentration of 0:1 M and a final pH of 8 in the presence of either 5 mM CaCl₂ or 2.5 mM each of CaCl₂ and MgCl₂. The activation mixtures were incubated for 8 h (Fig. 2A) or 16 h (Fig. 2B) at about 4°C or about 20°C as indicated.

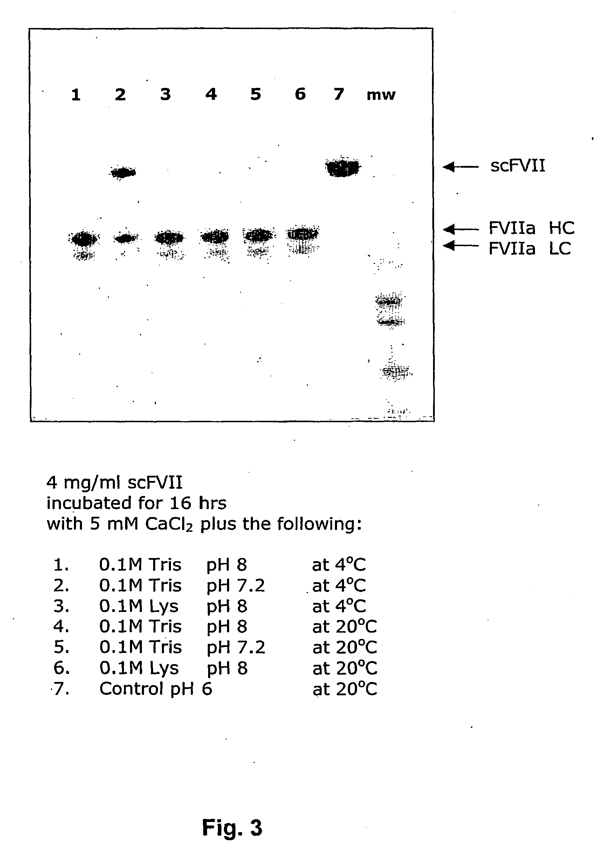

Figure 3 shows in-solution activation to FVIIa of a substantially purified preparation of scFVII. To the 4 mg/ml scFVII preparation (shown in lane 7) either Tris or lysine was added to a final total concentration of 0.1 M in the presence of 5 mM CaCl₂ and a final pH of either 7.2 or 8, as indicated. The activation mixtures were incubated for 16 h at about 4°C or about 20°C as indicated.

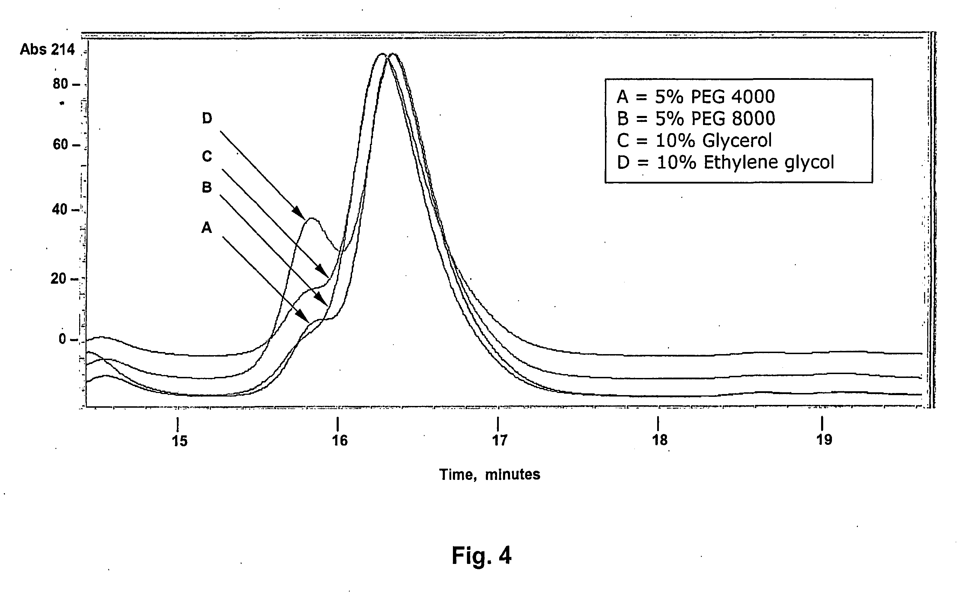

Figure 4 shows an RP-HPLC trace of the FVIIa peak following in-solution activation reactions in the presence of various activation enhancers. Activation of 3.5 mg/ml scFVII was carried out for 16 hrs at about 4°C in the presence of 0.1 M Tris, 5 mM CaCl₂ pH 8, and activation enhancers PEG 4000, PEG 8000, glycerol and ethylene glycol as indicated. The reaction products were analyzed by RP-HPLC on a C8 column, 2.1mm diameter x 150 mm length, 300 A°, 5mm from Grace Vydac. Mobile phase A was 0.1% TFA, 30% acetonitrile, and Mobile phase B was 0.1 % TFA, 80 % acetonitrile. The column was equilibrated in 30% acetonitrile, 0.1% TFA at 0.2 ml/min at a column temperature of 70 °C and scFVII or FVIIa samples were resolved using a gradient from 20-60% B over 37 min at 0.2 ml/min with detection at 214nm.

### DETAILED DESCRIPTION OF THE INVENTION

### DEFINITIONS

In the description and claims below, the follow definitions apply:
The term "FVII zymogen", or "scFVII" refers to a FVII molecule provided in single-chain form.

The term "FVIIa" refers to a FVII molecule provided in its activated two-chain form, wherein the peptide bond between R152 and I153 of the single-chain form has been cleaved.

The terms "rFVII" and "rFVIIa" refer to FVII and FVIIa molecules, respectively, produced by recombinant techniques. These may have the wild-type human sequence or may be variants of the human sequence.

The terms "hFVII" and "hFVIIa" refer to wild-type human FVII and FVIIa, respectively.

Unless it is indicated otherwise or is apparent from the context, the terms "FVII", "FVII protein" and "Factor VII" as used herein are intended to include the single-chain and activated forms of FVII, and to include the recombinant wild-type sequence of human FVII as well as variants thereof.

The term "autoactivation" refers to the activation of FVII to FVIIa without addition of another protease such as Factor XIIa or other protease with trypsin-like specificity.

A "substantially purified preparation of scFVII" predominantly comprises scFVII relative to FVIIa or other FVII-derived fragments; that is, comprises at least 80% scFVII and less than 20% FVIIa or other FVII-derived fragments. The substantially purified preparation of scFVII may comprise, e.g., at least 85%, at least 90%, at least 92%, at least 94%, at least 95%, at least 96%, at least 97%, at least 98% or at least 99% scFVII, relative to FVIIa or other FVII-derived fragments in the preparation. The amount of scFVII, FVIIa and other FVII-derived fragments in a preparation, in a solution or in a mixture may determined by methods known in the art, such as, quantitation of bands on electrophoretic gels or western blots by, e.g., visual inspection or densitometry; spectrophotometric detection and quantitation of peaks eluting from an analytical separation column; and the like. A substantially purified preparation of scFVII often gives rise to essentially one band on a reducing SDS-PAGE gel (see, e.g., Fig. 1).

An "activation enhancer" is a compound which accelerates the activation of scFVII when scFVII is present at low concentrations (e.g., less than about 4 mg/ml, such as above about 0.5 mg/ml and below about 4 mg/ml) in the activation mixture. Such activation enhancers include, for example, polyethylene glycol, glycerol, and ethylene glycol.

### IN-SOLUTION ACTIVATION OF FVII

FVII protein that may be activated by the method of the invention includes human recombinant FVII and variants thereof. Variants that may be activated by the method of the invention include, for example, those described in WO 01/58935, WO 03/093465, WO 2004/029091, WO 2004/111242, WO 99/20767, WO 00/66753, WO 88/10295, WO 92/15686, WO 02/29025, WO 01/70763, WO 01/83725, WO 02/02764, WO 02/22776, WO 02/38162, WO 02/077218, WO 03/027147, WO 03/037932, WO 2004/000366, WO 2004/029090, WO 2004/108763, and US 20050164932. The FVII protein may be produced, for example, in eukaryotic cells such as in mammalian or yeast cells, more preferably in mammalian cells such as CHO cells, HEK cells or BHK cells.

The FVII variant may include one or more substitutions, insertions or deletions compared to wild-type human FVII (SEQ ID NO: 1), for example resulting in a variant that differs in 1-15 amino acid residues from the amino acid sequence of wild-type human FVII, typically in 1-10 or in 2-10 amino acid residues, e.g. in 1-8 or in 2-8 amino acid residues, such as in 3-7 or in 4-6 amino acid residues from the amino acid sequence, where the differences in amino acid sequence from the wild-type are typically substitutions. Such substitutions may be performed e.g. with the aim of introducing one or more *in vivo* glycosylation sites or PEGylation sites into the protein and/or for improving or otherwise modifying the clotting activity of the wild-type protein; such variants are described, for example, in WO 01/58935, WO 03/093465, WO 2004/029091, WO 2004/111242 and US 20050164932. In one embodiment, the FVII variant comprises one or more of the substitutions P10Q, K32E, and A34E. In another embodiment, the FVII variant comprises the substitutions P10Q and K32E. In another embodiment, the FVII variant comprises one or more of the the substitutions T106N or V253N. In another embodiment, the FVII variant comprises the substitutions P10Q, K32E, T106N and V253N. In another embodiment, the FVII variant comprises the substitutions P10Q, K32E, A34E, T106N and V253N. In another embodiment, the FVII variant comprises the substitutions P10Q, K32E, A34E, R36E, T106N and V253N.

A solution comprising a substantially purified preparation of scFVII, containing at least 80% scFVII relative to FVIIa or other FVII-derived fragments, such as, e.g., at least 85%, at least 90%, at least 92%, at least 95%, or at least 98% scFVII relative to FVIIa or other FVII-derived fragments may be obtained according to the method described below, or by any other method known to those of ordinary skill in the art. To this solution the the following is added:
An amine compound, such as Tris, lysine, arginine, phosphorylcholine, or betaine, is added to a final concentration of about 50 mM to about 500 mM, such as about 100 mM.

Ca²⁺ (generally CaCl₂) is added to a final concentration of at least 2 mM, preferably about 5 mM to 50 mM, such as about 10 mM to about 30 mM.

The final pH of the solution is adjusted to between about pH 7.2 to 8.6, e.g., to about pH 7.4 to 8.4, such as about pH 7.6 to 8.2, e.g. about pH 8. If Tris, lysine, arginine, or phosphorylcholine are used in the activation mixture, an additional buffer is often not necessary to adjust the pH to to the desired pH. If betaine is used, which is not buffering, an additional buffer is included to adjust the pH to the desired pH. The additional buffer need not be an amine-containing buffer; for example, borate is a suitable buffer. The concentration of NaCl in the activation mixture is generally between about 50 and 300 mM, preferably about 100 mM.

The resulting activation mixture is incubated between about 2°C and about 25°C, for example at "cold-room" temperature (e.g., about 2°C to 8°C, such as about 4°C) or at room temperature (e.g., about 18°C to 25°C, such as about 20°C or about 22°C), for an amount of time sufficient to convert at least 90% of the scFVII to FVIIa. Conversion of scFVII to FVIIa may be monitored for example by gel electrophoresis on reducing SDS-PAGE gels as descibed below, by size exclusion chromatography, or by any other means known to those of ordinary skill in the art.

The initial concentration of scFVII in the activation mixture is generally at least 0.5 mg/ml, such as at least 1 mg/ml, e.g., at least 4 mg/ml, such as, for example, between about 4 mg/ml and 10 mg/ml. If the activation mixture has an initial concentration of scFVII less than 4 mg/ml, the activation mixture preferably further comprises an activation enhancer, such as polyethylene glycol, glycerol, or ethylene glycol, such as, for example, PEG4000, PEG8000, or glycerol. The PEG4000 or PEG8000 is preferably present at between about 1% and 10% (w/v) in the activation mixture, such as, for example, 5% PEG4000 or 5% PEG8000. Glycerol or ethylene glycol is preferably present at between about 5% and 15% (v/v) in the activation mixture, such as, for example, 10% glycerol or 10% ethylene glycol, preferably 10% glycerol.

A major advantage of the in-solution activation method of the present invention over existing activation methods, such as those employing a positively charged surface or anion-exchange resin, is that the in-solution activation process is more amenable to optimization, for example at a small scale, and the parameters so optimized may be readily scaled up for large-scale manufacturing. Parameters for in-solution activation at a large scale may furthermore be more readily adjusted to adapt to process variations. Conditions can be established for the activation reaction to proceed over a timeframe and at a temperature practical for large-scale manufacturing purposes, such as, e.g, about 4 hrs, 8 hrs, overnight (e.g., 16 hrs), or 24 hours; preferably 4 hrs or 16 hrs, and, for example, either at "cold-room" temperature or at room temperature. The ability to carefully control the activation process furthermore minimizes the formation of undesirable degradation products.

The invention will be further described with reference to the following non-limiting example.

### MATERIALS AND METHODS

### PREPARATION OF scFVII

An exemplary FVII variant comprising the subsitutions P10Q K32E A34E R36E T106N V253N (relative to SEQ ID NO: 1) was expressed in CHO-K1 cells and FVII variant protein was secreted into the culture media. The culture supernatant was sterile-filtered and ultrafiltered. The pH of the concentrated supernatants was adjusted to 6 with acetic acid, CaCl₂ concentration was adjusted to 0.25 mM, L-histidine concentration was adjusted to 10 mM, and Tween-80 was added to 0.04%.

The adjusted supernatant was subsequently loaded onto either an ANX Sepharose™ 4 FF column or a Q-Sepharose™ FF column (GE Healthcare), previously equilibrated with 25 mM L-histidine-HCl, 140 mM NaCl, 0.04% Tween-80 pH 6. The column was then washed with 25 mM L-histidine-HCl, 140 mM NaCl, 0.04% Tween-80 pH 6, followed by 25 mM L-histidine-HCl, 1 mM CaCl₂, 0.04% Tween-80 pH 6. The protein was eluted with either 25 mM L-histidine-HCl, 34 mM CaCl₂, 0.04% Tween-80 pH 6 when using ANX-Sepharose 4 FF, or 25 mM L-histidine-HCl, 33 mM CaCl₂, 0.04% Tween 80 pH 6 when using Q-Sepharose FF.

The solution eluted from the ANX- or the Q-Sepharose column was adjusted to a final concentration of 100 mM NaCl before being applied to a MAb-affinity column (prepared by the coupling a monoclonal calcium-dependent anti FVIIIG1a-domain antibody to CNBr-activated Sepharose FF) previously equilibrated with 25 mM L-histidine-HCl, 100 mM NaCl, 35 mM CaCl₂ pH 6. The column was washed with 25 mM L-histidine-HCl, 25 mM NaCl, 0.25 mM CaCl₂ pH 6, and scFVII was eluted from the column in 25 mM L-histidine-HCl, 25 mM NaCl, 5 mM EDTA pH 6. The scFVII eluted from the MAb column had a purity of about 95% as determined visually on an SDS-PAGE gel.

The substantially purified scFVII preparation was either dialyzed or diafiltered against 10 mM L-histidine-HCl, 120 mM NaCl, 0.25 mM CaCl₂ pH 6, optionally containing 0.01 % Tween-80, and the protein was further concentrated as desired. Under these conditions, the scFVII preparation was stable at room temperature (approx 20°C) for at least 24 hours in the presence of 0.25 mM to 50 mM CaCl₂ (Figure 1).

### EXAMPLE 1

Single-chain FVII (scFVII) prepared as described above was incubated at cold-room temperature (approximately 4°C, with a range of about 2°C to 8°C) or room temperature (approximately 20°C, with a range of about 18°C to 25°C) under various conditions. Conversion of scFVII to FVIIa was monitored by gel electrophoresis on reducing SDS-PAGE gels. The presence of any further degradation product was monitored using spectrophotometric quantitation of peaks eluted from a size exclusion chromatography (SEC) column or a reverse-phase HPLC (RP-HPLC) column.

The amine compounds Tris, lysine, arginine, phosphorylcholine, and betaine were effective in converting scFVII to FVIIa when added to the substantially purified scFVII preparation in the presence of at least 5 mM CaCl₂ at pH 8. Tris and lysine were found to be effective at concentrations between about 50 mM and 0.5 M in the presence of 5 mM CaCl₂ and pH 8. Arginine, phosphorylcholine, and betaine were effective at a final concentration of about 0.1 M in the presence of 5 mM CaCl₂ and pH 8. An additional buffering agent was generally not necessary to adjust the pH of the activation mixture to 8 when Tris, lysine, arginine, or phosphorylcholine were used. When betaine was employed, which is non-buffering, a buffer such as borate, HEPES or TRIS was added to the activation mixture to adjust the pH to 8. The concentration of NaCl in the activation mixture was generally about 100 mM.

The conversion of scFVII to FVIIa was sensitive to the concentration of Ca²⁺ in the activation mixture. Activation was minimal in the presence of 0.25 mM CaCl₂ but was robust in the presence of at least 2 mM CaCl₂, such as in the range of 5 mM CaCl₂ to 50 mM CaCl₂, e.g., 10 mM CaCl₂ to 30 mM CaCl₂. Mg²⁺ could substitute for at least part of the Ca²⁺, as demonstrated below.

There was a significant dependence of the initial concentration of scFVII on the time for activation. Figs. 2A and 2B show the timecourse for in-solution activation of an initial concentration of 8 mg/ml scFVII (shown in lane 9 of Fig. 2B). Fig. 2A shows that in the presence of 5 mM Ca²⁺ and either 0.1 M Tris or 0.1 M lysine at pH 8, conversion of 8 mg/ml scFVII to FVIIa was essentially complete after 8 hrs at room temperature (RT; about 20°C, lanes 1 and 2) and was about 95% complete after 8 hrs at about 4°C (lanes 3 and 4). Under these conditions, in-solution activation in the presence of 2.5 mM Ca²⁺ plus 2.5 mM Mg²⁺ proceeded slightly slower than in the presence of 5 mM Ca²⁺ (lanes 5-9). After 16 hrs (Fig. 2B), conversion of 8 mg/ml scFVII to FVIIa was essentially complete at both 4°C and RT, and at 5 mM Ca²⁺ and at 2.5 mM Ca²⁺ plus 2.5 mM Mg²⁺.

Fig. 3 shows that at an initial scFVII concentration of 4 mg/ml, conversion from scFVII to FVIIa at pH 8 and 5 mM Ca²⁺ in the presence of 0.1 M Tris or 0.1 M lysine was essentially complete after 16 hrs at RT (lanes 4 and 6), and was about 90% complete after 16 hrs at about 4°C (lanes 1 and 3). Fig. 3 also demonstrates the role of pH in the in-solution activation reaction; comparison of lanes 1 and 2, and of lanes 4 and 5, show that all other parameters being equal, activation at pH 7.2 was slower than activation at pH 8. Activation at pH to 8.6 was comparable to that at pH 8.0 (data not shown).

At initial concentrations of scFVII lower than about 4 mg/ml it was found that the presence of an activation enhancer such as PEG (e.g., PEG4000 or PEG8000) and glycerol was effective in accelerating the activation reaction, such that significant activation could be achieved in 16 hrs without generation of an unacceptable level of degradation products. Optimal results were observed using 5% (w/v) PEG4000, 5% (w/v) PEG8000, and 10% (v/v) glycerol (Fig. 4).

Thus, parameters such as scFVII concentration, concentration of amine compound, Ca²⁺ or Ca²⁺/Mg²⁺ concentration, pH, and temperature can be adjusted to obtain a desired timeframe for the activation reaction, which is particularly useful in large-scale manufacturing applications.

While the foregoing invention has been described in some detail for purposes of clarity and understanding, it will be clear to one skilled in the art from a reading of this disclosure that various changes in form and detail can be made without departing from the true scope of the invention. It is understood that the examples and embodiments described herein are for illustrative purposes only and that various modifications or changes in light thereof will be suggested to persons skilled in the art and are to be included within the purview of this application and scope of the appended claims. For example, all the techniques and apparatus described above may be used in various combinations.

### SEQUENCE LISTING

<110> Maxygen Holdings Ltd.
   Krebber, Claus M.
   Viswanathan, Sridhar
<120> IN-SOLUTION ACTIVATION OF FACTOR VII
<130> 0285wo210
<150> US 60/702,041
   <151> 2005-07-22
<160> 1
<170> FastSEQ for Windows Version 4.0
<210> 1
   <211> 406
   <212> PRT
   <213> Homo sapiens
<400> 1

## Claims

1. A method for activating FVII to FVIIa in solution, comprising
(a) obtaining a solution comprising a substantially purified preparation of scFVII;
(b) adding to the solution an amine compound, Ca²⁺ to a final concentration of about 2 mM to about 50 mM, and adjusting the final pH of the solution to about pH 7.2 to 8.6;
(c) incubating the resulting activation mixture at between about 2°C and about 25°C for an amount of time sufficient to convert at least 90% of the scFVII to FVIIa; and
(d) optionally, isolating the FVIIa from the activation mixture.

2. The method of claim 1 wherein the solution comprising the substantially purified preparation of scFVII contains at least 85 % scFVII relative to FVIIa or other FVII-derived fragments.

3. The method of claim 1 wherein the amine compound is Tris, lysine, arginine, phosphorylcholine, or betaine.

4. The method of claim 1 wherein the amine compound is added to a final concentration of about 50 mM to about 500 mM.

5. The method of claim 1 wherein the final pH of the solution is pH 7.6 to 8.2

6. The method of claim 5 wherein the final pH of the solution is about pH 8.

7. The method of claim 1 wherein the activation mixture has an initial concentration of scFVII of at least 4 mg/ml.

8. The method of claim 1 wherein the activation mixture has an initial concentration of scFVII of about 1. mg/ml to 4 mg/ml, and the activation mixture further comprises an activation enhancer.

9. The method of claim 8 wherein the activation enhancer is polyethylene glycol, glycerol, or ethylene glycol.

10. The method of claim 9 wherein the activation enhancer is PEG4000, PEG8000, or glycerol.

11. The method of claim 1 wherein the amount of time sufficient to convert at least 90% of the scFVII to FVIIa is between about 4 hours and 24 hours.

12. The method of claim 1 wherein the activation mixture has an initial concentration of scFVII of about 4 mg/ml to about 10 mg/ml, the amine compound is Tris or lysine at a final concentration of about 0.1 M, the Ca²⁺ concentration is 10 mM to 30 mM, the pH of the activation mixture is about pH 8, and the solution is incubated at about 4°C for 8-24 hrs.

13. The method of claim 1 wherein the FVII is a FVII variant which differs in 1-15 amino acid residues from the amino acid sequence of human FVII (SEQ ID NO:1).

14. The method of claim 13 wherein the FVII variant comprises the substitutions P10Q and K32E.

## Patentansprüche

1. Verfahren zum Aktivieren von FVII zu FVIIa in Lösung, das Folgendes umfasst:
(a) Gewinnen einer Lösung, die ein im Wesentlichen gereinigtes scFVII-Präparat umfasst;
(b) Versetzen der Lösung mit einer Aminverbindung, Ca²⁺ auf eine Endkonzentration von ungefähr 2 mM bis ungefähr 50 mM, und Einstellen des End-pH-Werts der Lösung auf ungefähr pH 7,2 bis 8,6;
(c) Inkubieren der erhaltenen Aktivierungsmischung bei zwischen ungefähr 2°C und ungefähr 25°C über einen Zeitraum, der ausreicht, um mindestens 90% des scFVII in FVIIa umzuwandeln; und
(d) gegebenenfalls Isolieren des FVIIa aus der Aktivierungsmischung.

2. Verfahren nach Anspruch 1, wobei die Lösung, die das im Wesentlichen gereinigte scFVII-Präparat umfasst, mindestens 85% scFVII in Bezug auf FVIIa oder andere von FVII abstammende Fragmente enthält.

3. Verfahren nach Anspruch 1, wobei es sich bei der Aminverbindung um Tris, Lysin, Arginin, Phosphorylcholin oder Betain handelt.

4. Verfahren nach Anspruch 1, wobei die Aminverbindung auf eine Endkonzentration von ungefähr 50 mM bis ungefähr 500 mM zugesetzt wird.

5. Verfahren nach Anspruch 1, wobei der End-pH-Wert der Lösung pH 7,6 bis 8,2 beträgt.

6. Verfahren nach Anspruch 5, wobei der End-pH-Wert der Lösung ungefähr pH 8 beträgt.

7. Verfahren nach Anspruch 1, wobei die Aktivierungsmischung eine anfängliche scFVII-Konzentration von mindestens 4 mg/ml aufweist.

8. Verfahren nach Anspruch 1, wobei die Aktivierungsmischung eine anfängliche scVII-Konzentration von ungefähr 1 mg/ml bis 4 mg/ml aufweist und die Aktivierungsmischung weiterhin einen Aktivierungsverstärker umfasst.

9. Verfahren nach Anspruch 8, wobei es sich bei dem Aktivierungsverstärker um Polyethylenglykol, Glycerin oder Ethylenglykol handelt.

10. Verfahren nach Anspruch 9, wobei es sich bei dem Aktivierungsverstärker um PEG4000, PEG8000 oder Glycerin handelt.

11. Verfahren nach Anspruch 1, wobei der Zeitraum, der ausreicht, um mindestens 90% des scFVII in FVIIa umzuwandeln, zwischen ungefähr 4 Stunden und 24 Stunden beträgt.

12. Verfahren nach Anspruch 1, wobei die Aktivierungsmischung eine anfängliche scFVII-Konzentration von ungefähr 4 mg/ml bis ungefähr 10 mg/ml beträgt, es sich bei der Aminverbindung um Tris oder Lysin in einer Endkonzentration von ungefähr 0,1 M handelt, die Ca²⁺-Konzentration 10 mM bis 30 mM beträgt, der pH-Wert der Aktivierungsmischung ungefähr pH 8 ist und die Lösung 8-24 Stunden lang bei ungefähr 4°C inkubiert wird.

13. Verfahren nach Anspruch 1, wobei es sich bei dem FVII um eine FVII-Variante, die sich von der Aminosäuresequenz von Human-FVII (SEQ ID NO: 1) in 1-15 Aminosäureresten unterscheidet, handelt.

14. Verfahren nach Anspruch 13, wobei die FVII-Variante die Substitutionen P10Q und K32E umfasst.

## Revendications

1. Procédé pour l'activation de FVII en FVIIa en solution, comprenant
(a) l'obtention d'une solution comprenant une préparation en grande partie purifiée de scFVII ;
(b) l'ajout à la solution d'un composé amine, de Ca²⁺ à une concentration finale d'environ 2 mM à environ 50 mM et l'ajustement du pH final de la solution à environ pH 7,2 à 8,6 ;
(c) l'incubation du mélange d'activation résultant à une température comprise entre environ 2 °C et environ 25 °C pendant une durée suffisante pour convertir au moins 90 % du scFVII en FVIIa ; et
(d) facultativement, l'isolement du FVIIa du mélange d'activation.

2. Procédé selon la revendication 1 dans lequel la solution comprenant la préparation en grande partie purifiée de scFVII contient au moins 85 % de scFVII par rapport au FVIIa ou d'autres fragments dérivés de FVII.

3. Procédé selon la revendication 1 dans lequel le composé amine est le Tris, la lysine, l'arginine, la phosphorylcholine ou la bétaïne.

4. Procédé selon la revendication 1 dans lequel le composé amine est ajouté à une concentration finale d'environ 50 mM à environ 500 mM.

5. Procédé selon la revendication 1 dans lequel le pH final de la solution est pH 7,6 à 8,2.

6. Procédé selon la revendication 5 dans lequel le pH final de la solution est environ pH 8.

7. Procédé selon la revendication 1 dans lequel le mélange d'activation a une concentration initiale en scFVII d'au moins 4 mg/ml.

8. Procédé selon la revendication 1 dans lequel le mélange d'activation a une concentration initiale en scFVII d'environ 1 mg/ml à 4 mg/ml et le mélange d'activation comprend en outre un amplificateur d'activation.

9. Procédé selon la revendication 8 dans lequel l'amplificateur d'activation est le polyéthylèneglycol, le glycérol ou l'éthylèneglycol.

10. Procédé selon la revendication 9 dans lequel l'amplificateur d'activation est le PEG4000, le PEG8000 ou le glycérol.

11. Procédé selon la revendication 1 dans lequel la durée suffisante pour convertir au moins 90 % du scFVII en FVIIa est comprise entre environ 4 heures et 24 heures.

12. Procédé selon la revendication 1 dans lequel le mélange d'activation a une concentration initiale en scFVII d'environ 4 mg/ml à environ 10 mg/ml, le composé amine est le Tris ou la lysine à une concentration finale d'environ 0,1 M, la concentration en Ca²⁺ est de 10 mM à 30 mM, le pH du mélange d'activation est environ pH 8 et la solution est incubée à environ 4 °C pendant 8-24 h.

13. Procédé selon la revendication 1 dans lequel le FVII est un variant de FVII qui diffère en ce qui concerne 1-15 résidus d'acides aminés de la séquence d'acides aminés de FVII humain (SEQ ID n° : 1).

14. Procédé selon la revendication 13 dans lequel le variant de FVII comprend les substitutions P10Q et K32E.
